# EUROPEAN PATENT SPECIFICATION

(11) **EP 0 439 516 B1**
(45) Date of publication and mention of the grant of the patent: **11.01.1995**
(21) Application number: 89912075.2
(22) Date of filing: 23.10.1989
(51) Int. Cl.: A61B 17/34

(54) **INTRAOSSEOUS NEEDLE ASSEMBLY**
NADELEINHEIT FÜR KNOCHENINNENRAUM
ENSEMBLE D'AIGUILLE INTRA-OSSEUSE

(30) Priority: 24.10.1988 US 261699
(43) Date of publication of application: 07.08.1991
(73) Proprietor: Cook Incorporated, Bloomington Indiana 47402 (US); UNIVERSITY OF FLORIDA RESEARCH FOUNDATION, INC., Alachua, FL 32615 (US)
(72) Inventor: MELKER, Richard, J., Gainesville, FL 32605 (US); GEAREN, Peter, F., Gainesville, FL 32601 (US); MILLER, Gary, J., Gainesville, FL 32601 (US); DeBRUYNE, Michael, P., Bloomington, IN 47408 (US); MOLITOR, Lisa, Gainesville, FL 32601 (US)
(74) Representative: Bannerman, David Gardner
(86) International application number: US8904715
(87) International publication number: WO9004364

(56) References cited:
- DE-C- 265 433
- FR-A- 2 138 152
- GB-A- 2 130 890
- US-A- 2 525 329
- US-A- 3 750 667

## Description

The present invention relates to infusion needles, and more particularly, to an intraosseous infusion needle assembly having a tip adapted to bore directly into the patient's bone.

In a variety of medical emergencies, the patient's life may hinge upon the ability of the physician or medical attendant to administer a particular fluid into the patient's bloodstream. In emergency situations such as on the battlefield, at traffic accident scenes or in the emergency room, the patient is often in shock, has low blood pressure, is bleeding profusely and may be thrashing about. Under such circumstances, finding and gaining access to a suitable blood vessel can be all but impossible, the resulting delay in administering drugs to the patient possibly being fatal. In the case of children or infants in any emergency, even the largest veins are so small that they may not be located. Even if located, an infant's largest available vein may be so small that stable infusion access may not be possible.

One alternative to venous access, recently reintroduced, is the intraosseous route. The medullary cavity of the long bones is composed of a rich spongy network of venous sinusoids which drain into a central venous canal. Blood then exits the venous canal by nutrient or emissary veins into the circulation. Fluids or drugs injected into the medullary area usually diffuse only a few centimeters, enter the bloodstream and reach the heart--all in only about 10 seconds from injection into the medullary cavity. The current intraosseous infusion procedure uses a hollow needle having a beveled top and a trocar or stylet. With the stylet telescopically positioned within and extending partially out the bevelled end of the needle, the needle and trocar assembly is forceably and perpendicularly advanced against and through the desired bone until the cortex has been punctured and the needle and trocar tip has advanced into the medullary space. The trocar is withdrawn, leaving the open end of the needle directly in the rich vascular network. Various complications, however, have made intraosseous infusion a less than ideal option. Although the needle and trocar assembly have a sharp, pointed tip, the medullary cavity may not be able to be penetrated under normal pressure. Too much force in trying to puncture the bone sometimes results in a bent needle, a broken needle, splintering of the bone, sliding off the bone and puncturing adjacent tissue or, more commonly, the needle is accidentally forced through the opposite side of the bone. If the needle is properly inserted into the medullary cavity, movements by the patient can easily dislodge the needle or cause it to be moved so that the end opening is occluded. These complications commonly arise in cases involving intraosseous infusion of infants and children. For persons older than six, the bones are too hard to successfully perform intraosseous infusion without realizing an extremely high incidence of the above complications. The procedure has therefore typically been limited to children less than six years old and only after several attempts have been made to achieve venous infusion.

US Patent No.3750667 describes an intraosseous needle having a threaded shaft with a passageway extending from its first end towards its second end, and boring means extending from the second end to initiate penetration into bone.

What is needed is an intraosseous infusion device which decreases the incidence and severity of the above described complications, which is easier to insert and which is more stable once inserted.

Generally speaking, there is provided an intraosseous needle assembly which allows for precise control and placement of the needle during intraosseous infusion procedures.

According to the invention we provide an intraosseous needle comprising:
a threaded shaft having first and second opposing ends and a passageway extending from the first end toward the second end;
boring means for initiating penetration of said shaft into bone, said boring means extending forwardly from the second end;
characterised in that the boring means is rigidly connected to the second end and in that said shaft has at least one opening proximal to the second end and in communication with the passageway. The boring means is preferably a solid, pointed tip having a pair of concave indentations which define four cutting edges, so that the tip is adapted for rapid and precise boring into the bone of adults, children or infants. The shaft preferably defines a pair of side ports, one located in the valley between the leading or first full thread and the second thread and the other side port located between the second thread and the third thread. The side ports are preferably located 90° apart and are both in communication with the passageway of the shaft. A handle in the shape of a ball knob may be adapted for telescopic and gripping connection to the trailing end of the needle and both the handle and the trailing end of the needle are preferably equipped with mutually engaging, torque-transmitting faces.

One intraosseous needle assembly according to the invention will now be described by way of example and with reference to the accompanying drawings:
FIG.1 is a plan view of an intraosseous needle of an intraosseous assembly in accordance with the preferred embodiment of the present invention.
FIG.2 is a fragmented plan view of the needle of FIG.1 which has been rotated 90° about its axis.
FIG.3 is a plan view, partly in section, of a handle and intraosseous needle of the intraosseous needle assembly of the present invention.
FIG.4 is a front view of the handle of FIG.3.
FIG.5 is a front end view of the intraosseous needle of FIG.1.

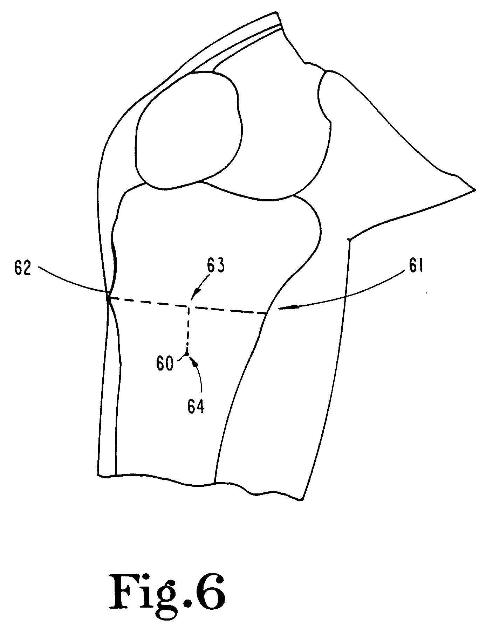

FIG.6 is a diagrammatic side view of the proximal tibia showing the preferred access site for the needle of the present invention.

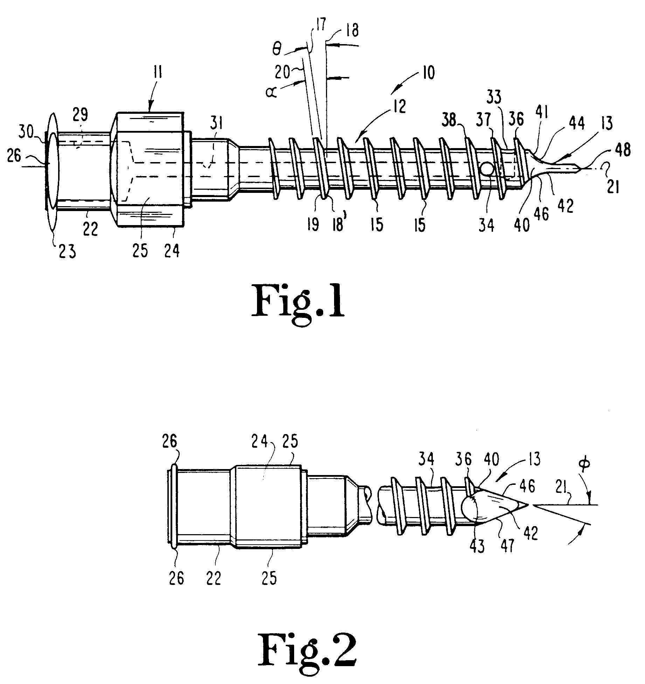

Referring now to FIGS.1 and 2 there is shown an intraosseous needle 10 in accordance with the preferred embodiment of the present invention. Needle 10 includes a hub 11, a threaded shaft 12 and boring means. In the preferred embodiment, the boring means is a fluted pencil point tip 13 and there are sixteen buttress threads 5.9 per cm (15 per inch) on shaft 12. The lead angle or helix angle ϑ is defined as the angle formed by a plane (indicated at 17) drawn tangent to the pitch helix and a plane (indicated at 18) normal to the axis 21 of threaded shaft 12. The leading and trailing thread surfaces are indicated at 18' and 19, respectively. The trailing thread angle α is defined here as that angle formed by a plane (indicated as 20) drawn tangent to trailing thread surface 19 and plane 18 normal to axis 12. Trailing thread angle α in the preferred embodiment is equal to the helix angle ϑ. That is, plane 17 is parallel to plane 20. Leading and trailing surfaces 18' and 19 are not parallel.

Hub 11, located at the rearward or trailing end of threaded shaft 12, forms the female end for connection to a conventional Luer-type fitting and includes a generally cylindrical portion 22 and an annular flange portion 23. A generally cylindrical section 24 is located between cylindrical portion 22 and threaded shaft 12. The diameters of flange portion 23 and cylindrical section 24 are approximately equal and both are greater than the diameter of cylindrical portion 22. Cylindrical section 24 has a pair of diametrically opposed and mutually parallel flat faces 25. Flange portion 23 likewise has a pair of diametrically opposed and mutually parallel flat faces 26 which are coplanar with corresponding flat faces 25. A large diameter bore 29 is defined in hub 11 and extends from end 30 through cylindrical portion 22 and partially through cylindrical section 24. Bore 29 receives the male portion of the Luer-type fitting. A smaller diameter axial passageway 31 is in communication with bore 29 and extends from bore 29 forwardly through nearly the entire length of threaded shaft 12. A pair of side ports 33 and 34 extend radially outwardly from axial passageway 31 near tip 13. Side ports 33 and 34 are located 90° apart. Side port 33 opens outwardly in the valley between the leading or first full thread 36 and the second thread 37. Side port 34 opens outwardly in the valley between the second thread 37 and the third thread 38.

Fluted pencil point tip 13 is substantially conical with the conical outer surface 40 forming an angle Φ with axis 21 of approximately 20°. A pair of diametrically opposed flutes 41 and 42 are milled into the end of tip 13 using a ball end mill. The end mill used to cut flutes 41 and 42 is aligned to rotate about an axis which is parallel with axis 21 during the milling process. The foremost end 43 of leading thread 36 is interrupted by the milling process such that leading thread 36 terminates into one of the flutes (42). The milling process thus forms sharp boring edges 44 and 45, between flute 41 and conical surface 40 and sharp boring edges 46 and 47 between flute 42 and conical surface 40. The border between leading thread 36 and flute 42 likewise forms a sharp cutting edge at 43. As shown in FIG. 1, flute 42 is machined further rearwardly than flute 41. In the preferred embodiment, the complete axial length of flute 41 measured from tip 48 is 0.36 cm (0.14 inches) while the complete axial length of flute 42 measured from tip 48 is 0.41 cm (0.16 inches).

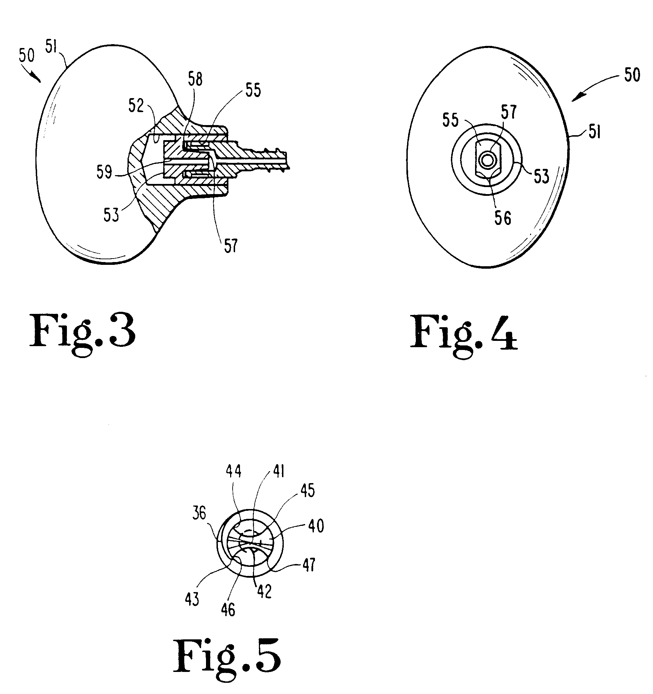

A complete intraosseous needle assembly includes, along with intraosseous needle 10, a corresponding gripping means or gripping element which is handle 50 (FIGS. 3 and 4). Handle 50 comprises a plastic ball knob 51 having an axial bore 52. An insert 53 for gripping needle 10 is sized to be tightly received within bore 52. Insert 53 is fixed within bore 52 by appropriate means such as by gluing. Insert 53 is adapted to couple with needle 10 and has a central opening 55 which is generally cylindrical with opposing planar faces 56. Opening 55 is sized to receive the complimentary shape of hub 11 with its cylindrical section 24 and opposing flat faces 25. Insert 53 further includes stub 57 which extends forwardly into opening 55. As hub 11 of needle 10 is received within opening 55, stub 57 enters bore 29 of hub 11. Stub 57 is tapered slightly forwardly such that its largest diameter, at its base 58, is the same as or just slightly larger than the inner diameter of bore 29. As stub 57 advances into hole 29, the larger diameter at base 58 of stub 57 wedges within bore 29 forming a snug fit between needle 10 and handle 50. Hub 11, bore 29, opening 55 and stub 57 are sized to create a mutually snug connection sufficient to cause hub 11 to remain firmly lodged within handle 50 but to be removed under a moderate manually applied tensile force.

Alternative embodiments are contemplated wherein the gripping means may be a permanently attached handle or other structure suitable for grasping by the person inserting the needle. The gripping means may also be or include some type of mechanical or electromechanical device which provides the necessary twisting and rotating action.

The intraosseous needle assembly is used as follows:
An intraosseous needle 10 is firmly secured to a handle 50. The preferred site is marked and an incision is made in the skin down to the bone. The preferred site 64 is found by first identifying the tibial tuberosity 62 on the anterior surface of the proximal tibia. An imaginery line is drawn from the tibial tuberosity to the median edge of the tibia 61. This line is equally divided 63 and the site of insertion 64 is perpendicular and distal to 63. The preferred site 64 increses in distance from 63 with increasing age. In the newborn or infant this distance may be as short as 0.3 - 0.5 cm and increases to approximately 2.5 cm by 6 years of age. Insertion at the level of the tibial tuberosity or distally, avoids insertion of the needle into the growth place of the tibia. The distal medial tibia is also an excellent site.

With handle 50 firmly in the palm of the operator's hand, the needle is selectively directed toward the desired access site 63 and contact with the bone is made. A back and forth twisting motion with slight pressure causes the four boring edges 44-47 to cut into the bone and enables flutes 41 and 42 to penetrate to the threaded section of needle 10. During the twisting motion, flutes 41 and 42 carry bone fragments out of the hole. Once the lead thread 36 reaches the hole 60, no further pressure is required. The operator simply screws the needle clockwise into the marrow to the desired depth. Obviously the size of the patient will determine how far to screw the needle in. As the needle is rotatably advanced, the design of threads 15 directs the marrow out and away from side ports 33 and 34. A fluid injected through needle 10 may then exit through side ports 33 and 34 unobstructed by marrow or other tissue which otherwise clogs conventional intraosseous needles.

With needle 10 in the desired position, handle 50 may be detached from needle 10 by slight, manually applied, tensile pressure therebetween. The appropriate drug administering mechanism such as a syringe or I.V. tubing may then be secured via the Luer-type fitting. After the patient has stabilized, venous access may be achieved and needle 10 may be removed by detaching the I.V. tubing or syringe from needle 10 and by resecuring handle 50 thereto. The needle may then be backed out by turning needle 10 counterclockwise.

## Claims

1. An intraosseous needle (10) comprising:
a threaded shaft (12) having first and second opposing ends and a passageway (31) extending from the first end toward the second end;
boring means (13) for initiating penetration of said shaft into bone, said boring means extending forwardly from the second end;
characterised in that the boring means is rigidly connected to the second end and in that said shaft has at least one opening (33) proximal to the second end and in communication with the passageway.

2. The intraosseous needle of claim 1 wherein said boring means (13) is generally conical with a pointed tip.

3. The intraosseous needle of claim 2 wherein said boring means (13) is solid and has at least two cutting edges (44,45).

4. The intraosseous needle of claim 3 wherein said at least one opening (29) is a side port located behind said boring means (13).

5. The intraosseous needle of claim 4 wherein said boring means (13) has a pair of concave indentations (41, 42) which define said at least two cutting edges (44,45).

6. The intraosseous needle of claim 5 wherein said shaft (12) has a plurality of threads (15), the thread nearest to said boring means being a leading thread (36) and wherein at least one of said indentations (41,42) interrupts the leading thread.

7. The intraosseous needle of claim 6 wherein there are two side ports (33,34) each of which is located between two adjacent threads (36 and 37, 37 and 38 respectively).

8. The intraosseous needle of claim 6 wherein said indentations define four cutting edges (44, 45, 46, 47) which enable said needle to cut into bone by rotating said needle in any direction about its axis.

9. The intraosseous needle of claim wherein each of said threads (15) has a helix angle(ϑ) and a trailing angle (α), the trailing thread angle and helix angle being substantially equal.

10. The intraosseous needle of claim 6 wherein said pair of concave indentations create four cutting edges (44,45,46,47), two of said four cutting edges for cutting upon rotation of said shaft in one direction about the axis and the other two of said four cutting edges for cutting upon rotation of said shaft in a direction opposite to the one direction.

11. An intraosseous needle assembly, comprising:
an intraosseous needle (10) having a threaded shaft (12) having first and second opposing ends and a passageway (31) extending from the first end toward the second end, boring means (13) extending forwardly from the second end for initiating penetration of said shaft into bone; and a first coupling means (11) at the first end for coupling with a gripping element (50), and
a gripping element (50) having a handle (51) and a second coupling means (55) for coupling with the first coupling means characterised in that the boring means (13) is rigidly connected to the second end and said shaft has at least one opening (33) proximal to the second end and in communication with the passageway.

12. The intraosseous needle assembly of claim 11 wherein said boring means is generally conical with a pointed tip.

13. The intraosseous needle assembly of claim 12 wherein said first (57) and second (55) coupling means both have mutually engageable torque transmitting means for transmitting torque between said gripping element and said needle.

14. The intraosseous needle assembly of claim 13 wherein said boring means (13) is solid and has at least two cutting edges.

15. The intraosseous needle assembly of claim 14 wherein said at least one opening is a first side port (33) located behind said boring means.

16. The intraosseous needle assembly of claim 15 wherein said shaft (12) has a plurality of threads (15), including a leading thread (36) proximal to said boring means, a second thread (37) adjacent to the leading thread, and a third thread (38) adjacent to the second thread, wherein said boring means has a pair of concave indentations (41,42) which define said at least two cutting edges (44,45,46,47) and wherein at least one of said indentations interrupts the leading thread (18).

17. The intraosseous needle assembly of claim 16 wherein there is a second side port (34) in communication with the passageway (12) and wherein said first side port (33) is located between the leading thread (36) and the second thread (37), wherein said second side port (34) is located between the second thread (37) and the third thread (38), and wherein said first and second side ports are 90° apart.

18. The intraosseous needle assembly of claim 17 wherein said torque transmitting means of said first coupling means is a generally cylindrical section (57) having diametrically opposed and mutually parallel planar faces, and wherein said torque transmitting means of said second coupling means is a generally cylindrical central opening (55) defining a pair of diametrically opposed and mutually parallel faces, said central opening being sized to telescopically receive said generally cylindrical section.

19. The intraosseous needle assembly of claim 18 wherein said handle (50) is an ovally-shaped ball knob (51).

20. An intraosseous needle according to claim 1 wherein said at least one opening proximal to the second end in communication with the passageway is a side port (33) located between two adjacent threads (36,37) of said shaft.

21. The intraosseous needle of claim 20 wherein there are two side ports (33,34) located on opposite sides of a portion of a thread of said shaft.

22. The intraosseous needle of claim 21 wherein the two side ports (33,34) are located approximately 90° apart.

## Patentansprüche

1. Knocheninnenraumnadel (10), welche aufweist:
einen mit Gewinde versehenen Schaft (12), der ein erstes und ein zweites Ende, die einander entgegengesetzt sind, sowie einen Durchgang (31) aufweist, der sich vom ersten Ende gegen das zweite Ende hin erstreckt:
eine Bohreinrichtung (13) zum Einleiten des Eindringens des genannten Schaftes in einen Knochen, wobei sich die genannte Bohreinrichtung von dem zweiten Ende nach vorn erstreckt;
dadurch gekennzeichnet, daß die Bohreinrichtung mit dem zweiten Ende fest verbunden ist und daß der genannte Schaft zumindest eine Öffnung (33) aufweist, die zu dem zweiten Ende benachbart und mit dem Durchgang in Verbindung ist.

2. Knocheninnenraumnadel nach Anspruch 1, bei der die genannte Bohreinrichtung (13) im wesentlichen konisch mit einer Endspitze ausgebildet ist.

3. Knocheninnenraumnadel nach Anspruch 2, bei der die genannte Bohreinrichtung (13) massiv ist und zumindest zwei Schneidkanten (44, 45) aufweist.

4. Knocheninnenraumnadel nach Anspruch 3, bei der die zumindest eine Öffnung (33) eine seitliche Durchtrittsöffnung ist, die hinter der genannten Bohreinrichtung (13) gelegen ist.

5. Knocheninnenraumnadel nach Anspruch 4, bei der die genannte Bohreinrichtung (13) ein Paar konkaver Auskehlungen (41, 42) aufweist, die die zumindest zwei Schneidkanten (44, 45) definieren.

6. Knocheninnenraumnadel nach Anspruch 5, bei der der genannte Schaft (12) eine Mehrzahl von Gewindewindungen (15) aufweist, von denen die der genannten Bohreinrichtung nächstgelegene Gewindewindung eine vordere Gewindewindung (36) ist, und wobei zumindest eine der genannten Auskehlungen (41, 42) die vordere Gewindewindung unterbricht.

7. Knocheninnenraumnadel nach Anspruch 6, bei der zwei seitliche Durchtrittsöffnungen (33, 34) vorhanden sind, von denen jede zwischen zwei benachbarten Gewindewindung (36 und 37 bzw. 37 und 38) gelegen ist.

8. Knocheninnenraumnadel nach Anspruch 6, bei der die genannten Auskehlungen vier Schneidkanten (44, 45, 46, 47) definieren, welche ermöglichen, daß die genannte Nadel durch Drehen der genannten Nadel in beliebigen Richtungen um ihre Achse in den Knochen einschneidet.

9. Knocheninnenraumnadel nach Anspruch, bei der jede der genannten Gewindewindungen (15) einen Schraubenwinkel (ϑ) und einen hinteren Winkel (α) besitzt, wobei der hintere Gewindewinkel und der Schraubenwinkel im wesentlichen gleich sind.

10. Knocheninnenraumnadel nach Anspruch 6, bei der das genannte Paar konkaver Auskehlungen vier Schneidkanten (44, 45, 46, 47) erzeugt, zwei der genannten vier Schneidkanten für das Schneiden bei Drehung des genannten Schaftes in einer Richtung um die Achse und die anderen zwei der genannten vier Schneidkanten für das Schneiden bei Drehung des genannten Schafts in einer zu der ersten Richtung entgegengesetzten Richtung.

11. Nadelanordnung für den Knocheninnenraum, welche aufweist:
eine Knocheninnenraumnadel (10) mit einem mit Gewinde versehenen Schaft (12), der ein erstes und ein zweites Ende, die einander entgegengesetzt sind, sowie einen Durchgang (31) aufweist, der sich von dem ersten Ende gegen das zweite Ende hin erstreckt, wobei sich eine Bohreinrichtung (13) von dem zweiten Ende nach vorn erstreckt, um das Eindringen des genannten Schaftes in einen Knochen einzuleiten, und mit einer ersten Kupplungseinrichtung (11) am ersten Ende für die Verbindung mit einem Griffelement (50), und wobei
das genannte Griffelement (50) eine Handhabe (51) sowie eine zweite Kupplungseinrichtung (55) für die Verbindung mit der ersten Kupplungseinrichtung aufweist,
dadurch gekennzeichnet, daß die Bohreinrichtung (13) mit dem zweiten Ende fest verbunden ist und daß der genannte Schaft zumindest eine Öffnung (33) aufweist, die dem zweiten Ende benachbart und mit dem Durchgang in Verbindung ist.

12. Nadelanordnung für den Knocheninnenraum nach Anspruch 11, bei der die genannte Bohreinrichtung im wesentlichen konisch mit einer Endspitze ausgebildet ist.

13. Nadelanordnung für den Knocheninnenraum nach Anspruch 12, bei der sowohl die genannte erste (57) als auch die genannte zweite (55) Kupplungseinrichtung je gegenseitig in Eingriff bringbare, drehmomentübertragende Mittel aufweisen, um ein Drehmoment zwischen dem genannten Griffelement und der genannten Nadel zu übertragen.

14. Nadelanordnung für den Knocheninnenraum nach Anspruch 13, bei der die genannte Bohreinrichtung (13) massiv ist und zumindest zwei Schneidkanten aufweist.

15. Nadelanordnung für den Knocheninnenraum nach Anspruch 14, bei der die genannte zumindest eine Öffnung eine erste seitliche Durchtrittsöffnung (33) ist, die hinter der genannten Bohreinrichtung gelegen ist.

16. Nadelanordnung für den Knocheninnenraum nach Anspruch 15, bei der der genannte Schaft (12) eine Mehrzahl von Gewindewindungen (15) aufweist, einschließlich einer der genannten Bohreinrichtung benachbarten vorderen Gewindewindung (36) und einer zweiten Gewindewindung (37), die der vorderen Gewindewindung benachbart ist, sowie einer der zweiten Gewindewindung benachbarten dritten Gewindewindung (38), wobei die genannte Bohreinrichtung ein Paar konkaver Auskehlungen (41, 42) aufweist, die die genannten zumindest zwei Schneidkanten (44, 45, 46, 47) definieren, und wobei zumindest eine der genannten Auskehlungen die vordere Gewindewindung (36) unterbricht.

17. Nadelanordnung für den Knocheninnenraum nach Anspruch 16, bei der eine zweite seitliche Durchtrittsöffnung (34) in Verbindung mit dem Durchgang (31) vorhanden ist und die genannte erste seitliche Durchtrittsöffnung (33) zwischen der vorderen Gewindewindung (36) und der zweiten Gewindewindung (37) gelegen ist, wobei die genannte zweite seitliche Durchtrittsöffnung (34) zwischen der zweiten Gewindewindung (37) und der dritten Gewindewindung (38) gelegen ist und die genannten ersten und zweiten Durchtrittsöffnungen um 90° zueinander versetzt sind.

18. Nadelanordnung für den Knocheninnenraum nach Anspruch 17, bei der die genannte drehmomentübertragende Einrichtung der genannten ersten Kupplungseinrichtung ein im wesentlichen zylindrischer Abschnitt (57) mit diametral einander gegenüberliegenden und zueinander parallelen ebenen Flächen ist und bei der die genannte drehmomentübertragende Einrichtung der genannten zweiten Kupplungseinrichtung eine im wesentlichen zylindrische zentrale Öffnung (55) ist, die ein Paar diametral einander gegenüberliegender und zueinander paralleler Flächen definiert, wobei die genannte zentrale Öffnung so dimensioniert ist, daß sie den genannten im wesentlichen zylindrischen Abschnitt teleskopartig aufnimmt.

19. Nadelanordnung für den Knocheninnenraum nach Anspruch 18, bei der die genannte Handhabe (50) ein oval geformter Kugelknauf (51) ist.

20. Knocheninnenraumnadel nach Anspruch 1, bei der die zumindest eine zu dem zweiten Ende benachbarte, in Verbindung mit dem Durchgang befindliche Öffnung eine seitliche Durchtrittsöffnung (33) ist, die zwischen zwei benachbarten Gewindewindungen (36, 37) des genannten Schaftes gelegen ist.

21. Knocheninnenraumnadel nach Anspruch 20, bei der zwei seitliche Durchtrittsöffnungen (33, 34) vorhanden sind, die auf einander entgegengesetzten Seiten eines Teiles einer Gewindewindung des genannten Schaftes gelegen sind.

22. Knocheninnenraumnadel nach Anspruch 21, bei der die zwei seitlichen Durchtrittsöffnungen (33, 34) etwa um 90° zueinander versetzt sind.

## Revendications

1. Aiguille intraosseuse (10) comprenant :
un arbre fileté (12) ayant une première et une seconde extrémités opposées et un chemin de passage (31) s'étendant depuis la première extrémité vers la seconde extrémité ;
des moyens de forage (13) pour initier la pénétration dudit arbre dans l'os, lesdits moyens de forage s'étendant en avant à partir de la seconde extrémité ;
ladite aiguille étant caractérisée en ce que les moyens de forage sont rigidement reliés à la seconde extrémité et en ce que ledit arbre comporte au moins une ouverture (33) proximale vers la seconde extrémité et en communication avec le chemin de passage.

2. Aiguille intraosseuse selon la revendication 1 dans laquelle lesdits moyens de forage (13) sont généralement coniques avec une pointe épointée.

3. Aiguille intraosseuse selon la revendication 2 dans laquelle lesdits moyens de forage (13) sont pleins et comportent au moins deux arêtes coupantes (44, 45).

4. Aiguille intraosseuse selon la revendication 3 dans laquelle ladite au moins une ouverture (29) est un orifice latéral situé derrière lesdits moyens de forage (13).

5. Aiguille intraosseuse selon la revendication 4 dans laquelle lesdits moyens de forage (13) comportent une paire d'indentations concaves (41, 42) qui définissent lesdites au moins deux arêtes coupantes (44, 45).

6. Aiguille intraosseuse selon la revendication 5 dans laquelle ledit arbre (12) comporte une pluralité de filets (15), le filet le plus proche desdits moyens de forage étant un filet d'attaque (36) et dans laquelle au moins l'une desdites indentations (41, 42) interrompt le filet d'attaque.

7. Aiguille intraosseuse selon la revendication 6 dans laquelle il y a deux orifices latéraux (33, 34) chacun desdits orifices étant situé entre deux filets adjacents (36 et 37, 37 et 38, respectivement).

8. Aiguille intraosseuse selon la revendication 6 dans laquelle lesdites indentations définissent quatre arêtes coupantes (44, 45, 46, 47) qui permettent à ladite aiguille de pénétrer par découpe dans l'os lorsqu'on tourne ladite aiguille dans n'importe quelle direction autour de son axe.

9. Aiguille intraosseuse selon l'une quelconque des revendications précédentes, dans laquelle chacun desdits filets (15) présente un angle (Θ) d'hélice et un angle (α) de fuite, l'angle du filet de fuite et l'angle de l'hélice étant sensiblement egaux.

10. Aiguille intraosseuse selon la revendication 6 dans laquelle ladite paire d'indentations concaves créent quatre arêtes de découpe (44, 45, 46, 47), deux desdites quatre arêtes de découpe étant destinées à découper par rotation dudit arbre dans une direction autour de l'axe et les deux autres desdites quatre arêtes de découpe étant destinées à découper par rotation de l'arbre dans une direction opposée à la première direction.

11. Ensemble d'aiguille intraosseuse comprenant :
une aiguille intraosseuse (10) présentant un arbre fileté (12) comportant une première et une seconde extrémités opposées et un chemin de passage (31) s'étendant depuis la première extrémité vers la seconde extrémité, des moyens de forage (13) s'étendant en avant depuis la seconde extrémité pour initier la pénétration dudit arbre dans l'os et un premier moyen de couplage (11) à la première extrémité pour assurer le couplage avec un élément de saisie (50) et,
un élément de saisie (50) ayant un manche (51) et un second moyen de couplage (55) pour assurer le couplage avec ledit premier moyen de couplage, ledit ensemble étant caractérisé en ce que les moyens de forage (13) sont rigidement reliés à la seconde extrémité et ledit arbre comporte au moins une ouverture (33) proximale vers la seconde extrémité et en communication avec le chemin de passage.

12. Ensemble d'aiguille intraosseuse selon la revendication 11 dans lequel lesdits moyens de forage sont généralement coniques avec une pointe épointée.

13. Ensemble d'aiguille intraosseuse selon la revendication 12 dans lequel lesdits premier (57) et second (55) moyens de couplage comportent tous deux des moyens de transmission de couple mutuellement engageables pour transmettre un couple entre ledit élément de saisie et ladite aiguille.

14. Ensemble d'aiguille intraosseuse selon la revendication 13 dans lequel lesdits moyens de forage (13) sont pleins et comportent au moins deux arêtes de découpe.

15. Ensemble d'aiguille intraosseuse selon la revendication 14 dans lequel ladite au moins une ouverture constitue un premier orifice latéral (33) disposé en arrière desdits moyens de forage.

16. Ensemble d'aiguille intraosseuse selon la revendication 15 dans lequel ledit arbre (12) comporte une pluralité de filets (15), comprenant un filet d'attaque (36) proximal vers lesdits moyens de forage, un second filet (37) adjacent au filet d'attaque, et un troisième filet (38) adjacent au second filet, et dans lequel ensemble lesdits moyens de forage comportent une paire d'indentations concaves (41, 42) qui définissent lesdites au moins deux arêtes de découpe (44, 45, 46, 47) et dans lequel au moins une desdites indentations interrompt le filet d'attaque (18).

17. Ensemble d'aiguille intraosseuse selon la revendication 16 dans lequel il y a un second orifice latéral (34) en communication avec le chemin de passage (12) et dans lequel ledit premier orifice latéral (33) est disposé entre le filet d'attaque (36) et le second filet (37), tandis que ledit second orifice latéral (34) est situé entre le second filet (37) et le troisième filet (38), et dans lequel lesdits premier et second orifices latéraux sont décalés de 90°.

18. Ensemble d'aiguille intraosseuse selon la revendication 17 dans lequel lesdits moyens de transmission de couple desdits moyens de couplage sont constitués par une section (57) généralement cylindrique comportant des faces planes mutuellement parallèles et diamétralement opposées, et dans lequel lesdits moyens de transmission de couple desdits seconds moyens de couplage sont formés par une ouverture (55) centrale généralement cylindrique définissant une paire de faces mutuellement parallèles et diamétralement opposées, ladite ouverture centrale étant dimensionnée de façon à recevoir télescopiquement ladite section généralement cylindrique.

19. Ensemble d'aiguille intraosseuse selon la revendication 18 dans lequel ledit manche (50) est un bouton (51) en forme de boule ovalisée.

20. Aiguille intraosseuse selon la revendication 1 dans laquelle ladite au moins une ouverture proximale vers la seconde extrémité en communication avec le chemin de passage est formée par un orifice latéral (33) disposé entre deux filets adjacents (36, 37) dudit arbre.

21. Aiguille intraosseuse selon la revendication 20 dans laquelle il y a deux orifices latéraux (33, 34) disposés sur les côtés opposés d'une partie d'un filet dudit arbre.

22. Aiguille intraosseuse selon la revendication 21 dans laquelle les deux orifices latéraux (33, 34) sont disposés approximativement à 90° l'un de l'autre.
